# FASCICULE DE BREVET EUROPEEN

(11) **EP 0 927 027 B1**
(45) Date de publication et mention de la délivrance du brevet: **21.05.2003**
(21) Numéro de dépôt: 97938961.6
(22) Date de dépôt: 29.08.1997
(51) Int. Cl.: A61K 31/00, A61K 31/445, A61P 5/00, A61P 9/12

(54) **UTILISATION D'ANTAGONISTES 5-HT 4? DANS LA REGULATION DE LA SECRETION DE CORTICOSTEROIDES**
VERWENDUNG VON 5-HT4 ANTAGONISTEN ZUR REGULIERUNG DER CORTICOSTEROID-SEKRETION
USE OF 5-HT 4? ANTAGONISTS FOR REGULATING CORTICOSTEROID SECRETION

(30) Priorité: 30.08.1996 FR 9610639
(43) Date de publication de la demande: 07.07.1999
(73) Titulaire: Centre Hospitalier Universitaire de Rouen, 76031 Rouen Cedex (FR)
(72) Inventeur: LEFEBVRE, Hervé, F-76960 Notre-Dame-de-Bondeville (FR)
(74) Mandataire: Casalonga, Axel
(86) Numéro de dépôt international: FR9701537
(87) Numéro de publication internationale: WO98008493

(56) Documents cités:
- V.CONTESSE ET AL.: "Effect of a series of 5-HT4 receptor agonists and antagonists on steroid secretion by the adrenal gland in vitro" EUR.J.PHARMACOL., vol. 265, no. 1-2, 14 novembre 1994, pages 27-33, XP000673150
- V.CONTESSE ET AL.: "Activation of 5-Hydroxytryptamine4 Receptors Causes Calcium Influx in Adrenocortical Cells: Involvement of Calcium in 5-Hydroxytryptamine-Induced Steroid Secretion" MOLECULAR PHARMACOLOGY, vol. 49, no. 3, mars 1996, pages 481-493, XP000673272
- DE K. SOMMERS ET AL.: "Effects of metoclopramide and tropisetron on aldosterone secretion possibly due to agonism and antagonism at the 5-HT4 receptor" EUROPEAN JOURNAL OF CLINICAL PHARMACOLOGY, vol. 50, no. 5, juillet 1996, pages 371-373, XP000673274
- S.S.HEGDE ET AL.: "Peripheral 5-HT4 Receptors" FASEB J., vol. 10, no. 12, octobre 1996, pages 1398-1407, XP002030384

## Description

La présente invention concerne le traitement de pathologies liées à un dysfonctionnement surrénalien et notamment au dérèglement de la sécrétion de corticostéroïdes.

Les glandes surrénales sont composées de deux parties de structures et de rôles distincts : le cortex qui synthétise plusieurs hormones à partir du cholestérol sanguin appelées corticostéroïdes, et la médulla qui produit les catécholamines. Les corticostéroïdes sont quant à eux divisés en trois familles selon leur action biologique. Les minéralocorticoïdes sont représentés essentiellement par l'aldostérone dont le rôle est de participer au maintien de l'équilibre hydrominéral de l'organisme et de la pression artérielle. Les glucocorticoïdes, tels que le cortisol, exercent un effet hyperglycémiant important. La trosième partie est représentée par les androgènes, hormones anabolisantes.

Il est classiquement considéré que les sécrétions d'aldostérone et de cortisol sont majoritairement contrôlées par des facteurs sanguins de nature hormonale ou ionique. Des études in vitro ont mis en évidence une influence sur la production de ces corticostéroïdes par la sérotonine, un neurotransmetteur.

Des travaux fondamentaux ont permis de démontrer l'existence de récepteurs de la sérotonine au niveau du tissu surrénalien. Ces récepteurs spécifiques à la sérotonine ont été identifiés comme étant des récepteurs 5-HT₄ surrénaliens.

De nombreux travaux de recherche déjà publiés concernent l'intervention de récepteurs 5-HT₄ notamment dans la régulation de la motricité colique, du rythme cardiaque et de la vidange vésicale. Des modulateurs, agonistes ou antagonistes, de ces récepteurs 5-HT₄ font de ce fait l'objet d'un intérêt croissant pour leur application dans des indications découlant des travaux précités, à savoir la colopathie fonctionnelle, les troubles du rythme cardiaque et l'incontinence urinaire.

Dans certains dysfonctionnements de l'activité surrénalienne, la sécrétion des corticostéroïdes n'est plus régulée, conduisant à des pathologies telles que l'hyperaldostéronisme primaire, ou encore l'hypercorticisme (syndrôme de Cushing). L'hyperaldostéronisme primaire entraîne en outre une hypertension artérielle souvent résistante aux traitements anti-hypertenseurs classiques.

Ces pathologies correspondent à des situations où la sécrétion des corticostéroïdes est excessive et autonome. En d'autres termes, il est habituel de constater, dans ces situations, une diminution, voire une perte de la réceptivité du tissu surrénalien aux facteurs physiologiques classiques de régulation.

Par ailleurs, certains dysfonctionnements de l'organisme peuvent entraîner une stimulation de l'activité surrénalienne conduisant à une élévation du taux sanguin en corticostéroïdes. Dans le cas d'une augmentation du taux sanguin d'aldostérone, dont la sécrétion obéit néanmoins au mécanisme physiologique normal de production de cette hormone, on parle d'hyperaldostéronisme secondaire. Cette affection n'est pas sans incidence sur l'équilibre hydrominéral de l'organisme (pérénnisation des oedèmes notamment).

La demanderesse a maintenant découvert, de façon surprenante, que l'utilisation seule de molécules agissant en tant qu'antagonistes du récepteur 5-HT₄, permet de diminuer de façon significative le taux de corticostéroïdes tels que l'aldostérone sécrétés par des glandes surrénales malades. En effet, bien que les antagonistes soient supposés se fixer sur les récepteurs 5-HT₄ surrénaliens, il n'était pas évident qu'ils puissent avoir un effet bloquant notamment au cours de dysfonctionnements surrénaliens, ni qu'ils puissent agir dans des proportions telles que les taux d'aldostérone ou de cortisol, anormalement élevés, soient significativement diminués, la sécrétion de corticostéroïdes étant en temps normal principalement contrôlée par des hormones telles que l'angiotensine II et l'ACTH ou le potassium sanguin. Ces résultats ont amené la demanderesse à considérer l'utilisation des antagonistes du récepteur 5-HT₄ dans des situations où le taux sanguin des corticostéroïdes est pathologiquement élevé.

Selon une mise en oeuvre de l'invention, les antagonistes du récepteur 5-HT₄ sont utilisés dans le traitement et/ou la prévention de pathologies telles que l'hyperaldostéronisme primaire ou secondaire et l'hypercorticisme, ainsi que des affections qui découlent de ces pathologies.

L'hyperaldostéronisme primaire, qui se caractérise par une élévation spontanée des taux sanguins de base d'aldostérone sans obéir au mécanisme physiologique normal de production d'aldostérone, induit généralement une hypertension artérielle souvent résistante aux traitements hypertenseurs classiques. L'utilisation d'antagonistes 5-HT₄ selon l'invention a également pour but de traiter ou de prévenir cette hypertension artérielle liée à l'hyperaldostéronisme primaire.

L'hypertension artérielle en général est à la source des cardiopathies hypertensives, c'est-à-dire de complications cardiaques liées à cette hypertension. Il s'avère que le développement de ces complications cardiaques soit positivement corrélé au taux sanguin d'aldostérone quel que soit le mécanisme de l'hypertension artérielle. Le risque de complications cardiaques augmente avec ce taux sanguin d'aldostérone.

C'est pourquoi un autre objet de l'invention est l'utilisation d'antagonistes du récepteur 5-HT₄ pour la préparation de médicaments destinés au traitement de l'hypertension artérielle. Le contrôle du taux sanguin d'aldostérone permet de prévenir ou de traiter la cardiopathie hypertensive développée au cours d'hypertension artérielle quel qu'en soit le mécanisme.

Les médicaments préparés utilisant les antagonistes du récepteur 5-HT₄ peuvent se présenter sous des formes diverses de compositions pharmaceutiquement acceptables.

La quantité effective nécessaire au traitement ou à la prévention des pathologies décrites ci-dessus, dépend notamment de la nature et de la gravité des dysfonctionnements traités, ainsi que du poids du patient. A titre indicatif, on peut estimer qu'une dose unitaire d'antagonistes 5-HT₄ contienne cet agent dans une quantité comprise entre 0,1 et 300 mg. On administrera ces doses habituellement une à quatre fois par jour. La posologie moyenne pour un patient adulte peut être, par exemple, 1 à 600 mg de produit administré oralement ou 0,1 à 100 mg de produit administré par voie intraveineuse, sous-cutanée ou intramusculaire.

Ces compositions pharmaceutiques, outre les antagonistes 5-HT₄, contiennent un support pharmaceutiquement acceptable qui varie en fonction de leurs formes galéniques.

Les compositions peuvent se présenter sous forme de cachets, de capsules, de préparation liquide orale, de poudre, de granulés, de suspension ou solution injectable ou transfusable, sous forme de suppositoire ou toute autre forme appropriée pour son administration. Pour la préparation des compositions pharmaceutiques incorporant les antagonistes 5-HT₄, on peut utiliser tous types d'excipients ou d'additifs appropriés aux propriétés de l'antagoniste et au mode d'administration de la composition pharmaceutique. Ceci englobe notamment les excipients et additifs classiques habituellement utilisés en pharmacie.

Il est également envisageable d'utiliser les antagonistes 5-HT₄ selon l'invention, dans une composition pharmaceutique contenant conjointement un ou plusieurs autres agents actifs.

Parmi les antagonistes du récepteur 5-HT₄ utilisables dans le cadre de l'invention, on peut notamment citer les antagonistes 5-HT₄ décrits dans de nombreuses publications et référencés sous les numéros ICS 205930, DAU 6285, GR 113808, R 50595, SDZ 205557, RS 23597-190, SB 207266A. On peut également citer les antagonistes 5-HT₄ décrits dans les demandes de brevets WO 94/27987, WO 91/16045, EP 501322, WO 93/12785, WO 94/27965. D'autres antagonistes du récepteur 5-HT₄ décrits dans une abondante littérature-brevet et non-brevet, peuvent également être mis en oeuvre dans le cadre de l'invention.

### EXEMPLE

### Effet d'un antagoniste 5-HT₄ sur le taux d'aldostérone sécrété par un adénome de Conn.

Des extraits tissulaires humains produisant un taux anormalement élevé d'aldostérone sont maintenus en vie quelques heures dans un milieu de culture apportant tous les nutriments nécessaires au fonctionnement cellulaire. Ces extraits tissulaires sont soumis à une technique d'incubation dynamique appelée périfusion, plus amplement décrite dans Leboulenger et al., Gen. Comp. Endocrinol. 1978, 36 : 327-338 et Lefebvre et al., Neuroscience 1992, 47 : 999-1007, afin d'étudier l'effet d'un antagoniste 5-HT₄ sur la sécrétion d'aldostérone.

Les essais ont été effectués sur des tissus surrénaliens d'origine humaine, provenant de tumeurs produisant de l'aldostérone encore appelées adénomes de Conn.

Après dissection des tissus, les extraits de la tumeur sont préincubés dans 5 ml d'une solution Krebs-Ringer à 20°C. Cette solution Krebs-Ringer est composée de 120 mM de NaCl, 0,67 mM de MgSO₄, 5 mM de KCl, 1,2 mM de KH₂PO₄, 2,6 mM de CaCl₂, 22,5 mM de NaHCO₃, additionnée de 10 mM de glucose et d'1 g/l d'albumine de sérum bovin.

L'effet de l'antagoniste GR 113 808 (Glaxo) a été étudié en utilisant la technique de périfusion.

Les fragments à analyser sont rincés à trois reprises avec 5 ml de solution Krebs-Ringer et mélangés avec un support, Bio-Gel P₂ (Bio-Rad Laboratories, Richmond, CA, USA) et transférés dans des colonnes en polystyrène (diamètre intérieur de 10 mm). Chaque colonne de périfusion contient approximativement 20 mg de tissu surrénalien. Les colonnes de périfusion sont alimentées par une solution Krebs-Ringer avec un débit constant de 200 µl/min. à une température de 24°C. Le milieu de périfusion est gazé en continu avec un mélange à 95% d'oxygène et 5% de dioxyde de carbone.

Avant l'addition de toute substance réactive, les tissus surrénaliens sont stabilisés pendant 2 heures.

Au bout de ce temps de stabilisation, l'antagoniste GR 113 808 est introduit dans la solution Krebs-Ringer à une concentration de 10⁻⁷ M.

Des fractions du perifusat sont collectées toutes les 5 minutes et les échantillons congelés jusqu'à la mesure du taux d'aldostérone par une méthode de radioimmunologie.

On observe une diminution du taux d'aldostérone sécrété par l'adénome de Conn pouvant atteindre 60%, en réponse à l'effet de l'antagoniste GR 113 808..

## Revendications

1. Utilisation d'un antagoniste du récepteur 5-HT₄ pour la préparation d'un médicament destiné à traiter et/ou prévenir l'hyper-aldostéronisme primaire.

2. Utilisation d'un antagoniste du récepteur 5-HT₄ pour la préparation d'un médicament destiné à traiter et/ou prévenir l'hyper-corticisme.

3. Utilisation d'un antagoniste du récepteur 5-HT₄ pour la préparation d'un médicament destiné à traiter et/ou prévenir l'hyper-aldostéronisme secondaire.

4. Utilisation d'un antagoniste du récepteur 5-HT₄ pour la préparation d'un médicament destiné à traiter et/ou prévenir l'hyper-tension artérielle associée à l'hyperaldostéronisme.

5. Utilisation d'un antagoniste du récepteur 5-HT₄ pour la préparation d'un médicament destiné au traitement de l'hypertension artérielle.

## Patentansprüche

1. Anwendung eines Antagonisten des 5-HT₄-Rezeptors zur Herstellung eines Medikamentes, welches zur Behandlung und/oder Vorbeugung von primärem Hyperaldosteronismus bestimmt ist.

2. Anwendung eines Antagonisten des 5-HT₄-Rezeptors zur Herstellung eines Medikamentes, welches zur Behandlung und/oder Vorbeugung von Hypercorticoismus bestimmt ist.

3. Anwendung eines Antagonisten des 5-HT₄-Rezeptors zur Herstellung eines Medikamentes, welches zur Behandlung und/oder Vorbeugung von sekundärem Hyperaldosteronismus bestimmt ist.

4. Anwendung eines Antagonisten des 5-HT₄-Rezeptors zur Herstellung eines Medikamentes, welches zur Behandlung und/oder Vorbeugung von arterieller Hypertonie in Verbindung mit Hyperaldosteronismus bestimmt ist.

5. Anwendung eines Antagonisten des 5-HT₄-Rezeptors zur Herstellung eines Medikamentes, welches zur Behandlung von arterieller Hypertonie bestimmt ist.

## Claims

1. Use of a 5-HT₄ receptor antagonist for preparing a medicament intended for treating and/or preventing primary hyperaldosteronism.

2. Use of a 5-HT₄ receptor antagonist for preparing a medicament intended for treating and/or preventing hypercorticism.

3. Use of a 5-HT₄ receptor antagonist for preparing a medicament intended for treating and/or preventing secondary hyperaldosteronism.

4. Use of a 5-HT₄ receptor antagonist for preparing a medicament intended for treating and/or preventing high blood pressure linked to hyperaldosteronism.

5. Use of a 5-HT₄ receptor antagonist for preparing a medicament intended for the treatment of high blood pressure.
